(19) Europäisches Patentamt
European Patent Office
Office européen des brevets

(11) Numéro de publication: **0 028 186**
**B1**

# (12) FASCICULE DE BREVET EUROPEEN

(45) Date de publication du fascicule du brevet: **11.01.84**

(51) Int. Cl.[3]: **C 07 C 179/22**, C 11 D 3/39

(21) Numéro de dépôt: **80401484.3**

(22) Date de dépôt: **17.10.80**

(54) **Procédé de fabrication de compositions contenant des hydroperoxydes de mono- ou polyalkylarylsulfonates de métal alcalin, et application desdites compositions à la préparation de détergents.**

(30) Priorité: **26.10.79 FR 7926583**

(43) Date de publication de la demande: **06.05.81 Bulletin 81/18**

(45) Mention de la délivrance du brevet: **11.01.84 Bulletin 84/2**

(84) Etats contractants désignés: **AT BE CH DE GB IT LI LU NL SE**

(56) Documents cités:
**EP - A - 0 005 098**
**FR - A - 2 088 716**
**GB - A - 889 652**
**US - A - 2 829 158**

(73) Titulaire: **Société Chimique des Charbonnages, Tour Aurore Place des Reflets Cédex no 5, F-92080 Paris La Défense 2 (FR)**

(72) Inventeur: **Couderc, Pierre, 78, rue Emile Zola, F-62400 Bethune (FR)**
Inventeur: **Caux, Gérard, 45, rue des Marronniers, Aix Noulette F-62160 Bully les Mines (FR)**

**EP 0 028 186 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Procédé de fabrication de compositions contenant des hydroperoxydes de mono- ou polyalkylarylsulfonates de métal alcalin, et application desdites compositions à la préparation de détergents

La présente invention concerne un procédé de fabrication de compositions contenant des hydroperoxydes de mono- ou polyalkylarylsulfonates de métal alcalin et l'application desdites compositions à la préparation de détergents.

Elle a pour premier objet un procédé de fabrication en milieu aqueux de compositions contenant des hydroperoxydes de mono- ou polyalkylarylsulfonates de métal alcalin caractérisé en ce que:

a) dans une première étape, on traite un mono- ou polyalkylarylsulfonate de métal alcalin, de formule:

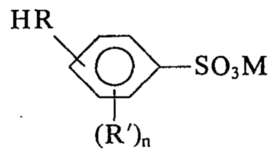

dans laquelle:
M est un métal alcalin, RH est un radical alkyle, de préférence isopropyle, R' est un radical alkyle inférieur tel que méthyle, éthyle, n-propyle, isopropyle, $n \leq 4$,
à l'aide d'oxygène, à une température inférieure à 85°C, à un pH compris entre 9 et 10,5, en présence de 1 à 50 ppm d'ion cobalt, par rapport au mono- ou polyalkylarylsulfonate de métal alcalin, pour former un mélange constitué de, en poids:

— 5 à 65% de mono- ou polyalkylarylsulfonate de métal alcalin, non transformé,
— 25 à 70% de l'hydroperoxyde correspondant, de formule:

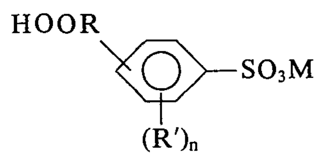

— 5 à 30% de l'alcool correspondant, de formule:

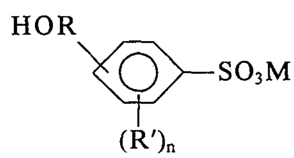

b) dans une deuxième étape, on met le mélange obtenu dans la première étape en contact avec de l'eau oxygénée, la quantité d'eau oxygénée étant comprise entre 2 et 15 parties en poids (comptées en eau oxygénée à 100%) pour 100 parties en poids du mélange obtenu dans la première étape, à un pH compris entre 4 et 10, à une température inférieure à 50° C, puis on sèche le produit ainsi traité à une température inférieure à 50° C.

Les mono- ou polyalkylarylsulfonates de métal alcalin utilisés comme matière première sont préparés, de façon connue en soi, par traitement de l'hydrocarbure correspondant par l'acide sulfurique concentré, à chaud, neutralisation par une base, refroidissement, filtration et séchage du précipité obtenu.

On peut ainsi utiliser comme matières premières, seuls ou en mélange, les sels alcalins des acides isopropylbenzènesulfonique (cumènesulfonique), diisopropyl-2,4-benzènesulfonique, méthyl-2-isopropyl-4-benzènesulfonique, méthyl-2-isopropyl-5-benzènesulfonique.

La première étape du procédé est grandement facilitée si l'on utilise comme catalyseur l'ion Cobalt apporté par le naphténate de cobalt. Le traitement de la première étape est effectué à l'aide d'oxygène pur ou d'un gaz inerte contenant de l'oxygène; on préfère utiliser l'air.

La solution aqueuse à traiter dans la deuxième étape du procédé contient le mélange résultant de la première étape selon une concentration quelconque par exemple comprise entre 30 et 70% en poids.

Le séchage prévu dans la deuxième étape du procédé selon l'invention se fera de préférence sous une pression comprise entre 2 et 100 mm de mercure.

Les composition selon la présente invention peuvent être associées à du tripolyphosphate de

sodium (TPP). En particulier il peut être avantageux d'effectuer la deuxième étape du procédé selon l'invention en ajoutant préalablement du TPP au mélange obtenu dans la première étape, le rapport pondéral $\frac{\text{TPP}}{\text{mélange}}$ étant par exemple compris entre 0,05 et 0,6. Dans ce cas le milieu réactionnel se trouve, de par l'effet tampon du TPP, à un pH de 9 à 9,5 et on obtient, après séchage, un produit dont l'augmentation relative du titre en oxygène actif est supérieure à celle que l'on obtient en absence de TPP.

Les compositions selon l'invention trouvent une utilisation intéressante comme agent de blanchiment, en particulier pour le lavage du linge, avec une efficacité accrue par rapport aux produits que l'on peut séparer après la première étape du procédé.

Le second objet de la présente invention consiste donc en des détergents contenant de 1 à 30% en poids d'une composition obtenue selon le procédé décrit ci-dessus.

Elles peuvent contenir également au moins un des ingrédients lessiviels connus : un tensio-actif anionique comme un alkylbenzènesulfonate de sodium, un savon tel que le sel de sodium de suif, un tensio-actif non ionique tel qu'un dérivé polyoxyéthyléné ou polyoxypropyléné, du tripolyphosphate de sodium, du silicate de sodium et du sulfate et/ou du carbonate de sodium.

Elles peuvent également contenir une quantité comprise entre 2 et 15% en poids d'un agent de blanchiment tel que le perborate ou le percarbonate de sodium.

Les détergents selon l'invention conduisent à des résultats au moins équivalents à ceux obtenus aves des détergents connus, bien que leur teneur en oxygène actif soit nettement plus faible.

Les exemples ci-dessous ont pour but d'illustrer, de façon non limitative, la présente invention.

## Exemple 1

Dans un réacteur tubulaire chauffé et agité, fonctionnant en circuit ouvert (donc sans recyclage de la phase gazeuse sortante), on introduit:

- 320 g de cumènesulfonate de sodium (1,44 mole),
- 10 ppm de Cobalt (par rapport au sulfonate) sous forme de naphténate de cobalt titrant 6% de Cobalt,
- 680 g d'eau,
- 1 g de $Na_2CO_3$,
- 6 g d'un mélange solide et sec de sulfonate, hydroperoxyde et alcool provenant d'une opération précédente et contenant 50% en poids d'hydroperoxyde du cumènesulfonate de sodium.

ö On injecte dans cette solution, dont le pH est de 9,5, un courant d'air à un débit de 10 litres/heure, et porte et maintient la température du réacteur à 80—82° C.

On arrête la réaction au bout de 32 heures. Le dosage des produits formés montre que la sélectivité de la réaction de peroxydation est de l'ordre de 80 à 85%. Cette solution contient 19% en poids d'hydroperoxyde du cumènesulfonate de sodium.

Le mélange réactionnel est séché à 40° C sous pression réduite jusqu'à obtention d'une poudre.

La poudre ainsi obtenue contient 51% en poids d'hydroperoxyde de cumènesulfonate de sodium, 20% de cumènesulfonate de sodium non transformé, 24% d'alcool provenant de la décomposition partielle de l'hydroperoxyde et 5% d'eau et sous-produits non identifiés. On dissout 50 g de cette poudre dans 50 g d'eau et amène le pH de la solution à 7. On ajoute à cette solution, à 25° C, 10 g d'eau oxygénée à 70% en poids (soit 14 parties d'eau oxygénée à 100% pour 100 parties de poudre) et on procède au séchage sous pression réduite à 20 mm de mercure, à une température maximale de 50° C.

On obtient ainsi une nouvelle poudre dont le titre, calculé en hydroperoxyde est de 59,6%. Le dosage, à l'aide de permanganate de potassium, de l'eau oxygénée libre contenue dans cette poudre permet de détecter la présence d'environ 2% d'eau oxygénée libre.

## Exemple 2

La peroxydation du cumènesulfonate de sodium, mis en solution aqueuse de même composition qu'à l'exemple 1, est réalisée de façon continue à l'aide d'air au débit de 10 l/h. Dans la phase de démarrage le réacteur est chargé avec la solution et l'air est injecté (température de peroxydation 80—82° C) jusqu'à ce que la solution contienne 18,6% d'hydroperoxyde du cumènesulfonate de sodium. A ce stade on introduit la solution de départ de façon continue au bas du réacteur et prélève par siphonnage un volume équivalent par la partie supérieure. Le temps de séjour dans le réacteur est d'environ 50 heures.

La solution ainsi prélevée et dont l'extrait sec (obtenu par séchage sous pression réduite à 40° C) est d'environ 35% en poids, est séchée partiellement sous vide à 40° C de façon à ce que sa teneur en hydroperoxyde soit amenée à 25% en poids, la teneur en matière sèche étant de 47% en poids.

A 100 g de cette solution, dont le pH est amené à 6,7, on ajoute 7,5 g d'eau oxygénée à 50% en poids, soit 8 parties d'eau oxygénée à 100% pour 100 parties de matière sèche, à 25° C. Le séchage complet de cette nouvelle solution à 45° C maximum sous pression réduite conduit à une poudre dont la teneur en oxygène actif, exprimée en hydroperoxyde du cumènesulfonate de sodium est de 58%.

Si le pH de la solution est amené, avant traitement par l'eau oxygénée, à une valeur de 5, on obtient, toutes conditions égales par ailleurs, une poudre dont le titre, exprimé en hydroperoxyde, est de 60% en poids. Si le pH est 9, le titre obtenu est de 56%.

## Exemple 3

100 g de la solution aqueuse provenant directement de la peroxydation selon l'exemple 2, titrant 18,6% d'hydroperoxyde et ayant un extrait sec de 35% est amenée à pH 5 puis traitée par 1,5 g d'eau oxygénée à 70% en poids (soit 3 parties d'eau oxygénée à 100% pour 100 parties de matière sèche). Après séchage à 40° C maximum, sous pression réduite à 20 mm de mercure, on obtient une poudre dont le titre exprimé en hydroperoxyde du cumènesulfonate de sodium est de 52%.

## Exemple 4

On ajoute à 100 g de solution obtenue par peroxydation selon l'exemple 1, ayant un extrait sec de 35%, et titrant 19% en poids d'hydroperoxyde, 20 g de tripolyphosphate de sodium. Le pH de la solution est 9,5. Après addition de 4,8 g d'eau oxygénée à 50% en poids à 25° C (soit 6,85 parties d'$H_2O_2$ à 100% pour 100 parties d'extrait sec, hors TPP), puis séchage à 40° C pendant 12 h sous pression réduite à 20 mm de mercure, on obtient 55 g d'une poudre dont le titre en oxygène actif, exprimé en hydroperoxyde du cumènesulfonate de sodium est de 42,5%.

## Exemples 5 à 9

Ces exemples concernent l'utilisation, en tant qu'agent de blanchiment, des compositions solides selon l'invention dans la préparation de détergents.

Les détergents sont réalisés par pesée des quantités voulues des divers ingrédients et dissous dans l'eau déminéralisée. On utilise pour le lavage un appareil de laboratoire de marque Ahiba »Texomat« type G6B, dont chaque bol est chargé de 6 g de poudre détergente et 600 ml d'eau. Le pH de la solution ainsi obtenue est ajusté à la valeur désirée par addition de traces de soude; la soude peut également être incorporée dans la poudre au cours de sa préparation par broyage. De préférence, la solution de lavage aura un pH compris entre 9 et 12 et mieux de 10±0,5. Dans tous les exemples ci-dessous il sera question du pH de la solution de détergent avant lavage, étant entendu que pendant le lavage, le pH dimunue d'environ 0,5 unité.

On chauffe jusque 30° et maintient 15 mn à cette température puis on introduit les paniers porte-tissus dans les bols et démarre l'agitation. On monte la température de 30 à 90—95° C pendant 40 mn et maintient pendant 50 mn à 90—95° C. Les paniers sont trempés dans l'eau courante et les échantillons de tissus rincés à l'eau déminéralisée puis séchés.

Les lavages sont effectués sur des échantillons de tissus-tests EMPA imprégnés des salissures artificielles suivantes : salissure normalisée (noir de carbone+graisse), vin rouge, noir Immédial (teinture au noir au soufre) que nous appellerons respectivement ci-dessous : S. N., vin, noir.

Les tissus comportant ces trois salissures sont lavés ensembles dans le même bol. L'efficacité du lavage par un détergent donné est mesurée à l'aide du systéme de chromaticité uniforme L, a, b, »cube root« (1958) tel que décrit dans la demande de brevet européen n° 5098.

Les tissus-tests utilisés dans les exemples ci-dessous ont, à l'origine, à l'état sali, les valeurs L, a, b, $\Delta E_{abs}$ suivantes

Tableau 1

| | L | a | b | $\Delta E_{abs}$ |
|---|---|---|---|---|
| S. N. | 45 | +2,5 | +1,9 | 55 |
| Vin | 71,7 | +9,5 | +6,9 | 31 |
| Noir | 70,6 | −0,1 | −1,9 | 30 |

Les détergents utilisés comprennent 73 g d'un mélange de base constitué de:

| | |
|---|---|
| Dodécylbenzènesulfonate de sodium à environ 50% de matière active | 10 g |
| Sel de sodium de suif | 7 g |
| Détergent non ionique | 6 g |
| Tripolyphosphate de sodium | 40 g |
| Silicate de sodium à environ 44% de matière active | 10 g |

A ce mélange de base on ajoute la composition solide obtenue selon l'exemple 2 et ayant une teneur en oxygène actif, exprimée en hydroperoxyde, de 58% et éventuellement du perborate de sodium selon les quantités indiquées au tableau 2, ainsi que 400 ppm d'ions $Cu^{++}$ (sous forme de $CuSO_4$, 5 $H_2O$), 0,05 g d'acide nitrilotriacétique et enfin on complète à 100 g par du carbonate de sodium.

Tableau 2

| | Exemple 5 | 6 | 7 | 8 |
|---|---|---|---|---|
| Produit peroxygéné selon l'ex. 2 | 10,2 | 10,2 | 25 | 4 |
| Peroborate de sodium | 2 | 4 | 0 | 10 |
| $Na_2CO_3$ | 15 | 13 | 2 | 13 |

Ces détergents conduisent aux résultats suivants, après lavage à pH 10–10,5 (ex. 5, 6, 8); 9,5–10 (ex. 7).

Tableau 3

| | | L | a | b | $\Delta E_{abs}$ |
|---|---|---|---|---|---|
| Ex. 5 | S. N. | 75,2 | 1,7 | 1,5 | 25 |
| | Vin | 91,6 | 1 | 3,4 | 9 |
| | Noir | 82,8 | 0,3 | −2,1 | 17 |
| Ex. 6 | S. N. | 76,1 | 2 | 0,4 | 24 |
| | Vin | 93,4 | 1,1 | −1,3 | 7 |
| | Noir | 83,4 | 0,5 | −4 | 17 |
| Ex. 7 | S. N. | 70 | 2,7 | 0,8 | 30 |
| | Vin | 88 | 3,3 | 3,7 | 13 |
| | Noir | 78 | 0,7 | −4,5 | 22 |
| Ex. 8 | S. N. | 73 | 2,3 | 1 | 27 |
| | Vin | 93 | 1,1 | −1,2 | 7 |
| | Noir | 83,5 | 0,4 | −4,1 | 17 |

Exemple 9 (comparatif)

A titre comparatif nous donnons ci-dessous les résultats obtenus à l'aide d'une poudre détergente commerciale contenant 25% en poids de perborate de sodium.

Tableau 4

| | L | a | b | $\Delta E_{abs}$ |
|---|---|---|---|---|
| S. N. | 74,7 | 1,9 | 0,8 | 25 |
| Vin | 93,3 | 0,8 | −1,7 | 7 |
| Noir | 83,3 | 0,2 | −4 | 17 |

**Revendications**

1. Procédé de fabrication en milieu aqueux de compositions contenant des hydroperoxydes de mono- ou polyalkylarylsulfonates de métal alcalin caractérisé en ce que:

a) dans une première étape, on traite un mono- ou polyalkylarylsulfonate de métal alcalin, de formule:

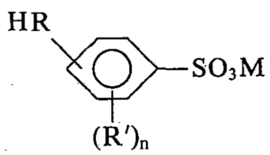

dans laquelle:
M est un métal alcalin, RH est un radical alkyle, R′ est un radical alkyle inférieur, $n \leq 4$,
à l'aide d'oxygène, à une température inférieure à 85° C, à un pH compris entre 9 et 10,5, en présence de 1 à 50 ppm d'ion cobalt, par rapport au mono- ou polyalkylarylsulfonate de métal alcalin, pour former un mélange constitué de, en poids:

- 5 à 65% de mono- ou polyalkylarylsulfonate de métal alcalin, non transformé,
- 25 à 70% de l'hydroperoxyde correspondant, de formule:

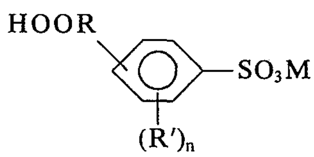

- 5 à 30% de l'alcool correspondant, de formule:

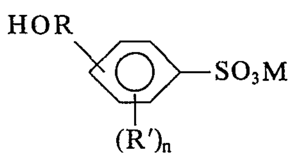

b) dans une deuxième étape, on met le mélange obtenu dans la première étape en contact avec de l'eau oxygénée, la quantité d'eau oxygénée étant comprise entre 2 et 15 parties en poids (comptées en eau oxygénée à 100%) pour 100 parties en poids du mélange obtenu dans la première étape, à un pH compris entre 4 et 10, à une température inférieure à 50° C, puis on sèche le produit ainsi traité à une température inférieure à 50° C.

2. Procédé selon la revendication 1 caractérisé en ce que l'ion cobalt est apporté par le naphténate de cobalt.

3. Procédé selon l'une des revendications précédentes caractérisé en ce que le traitement selon la première étape est réalisé à l'aide d'air.

4. Procédé selon l'une des revendications précédentes caractérisé en ce que la deuxieme étape du

procédé est réalisée sur une solution aqueuse contenant de 30 à 70% en poids de mélange obtenu dans la première étape.

5. Procédé selon l'une des revendications précédentes caractérisé en ce que le séchage du produit obtenu est effectué sous pression réduite entre 2 et 100 mm de mercure.

6. Procédé selon l'une des revendications précédentes caractérisé en ce que la deuxième étape est effectuée en présence de tripolyphosphate de sodium.

7. Procédé selon l'une des revendications précédentes caractérisé en ce que le mono- ou polyalkylarylsulfonate de sodium est l'isopropylbenzènesulfonate de sodium.

8. Application des compositions obtenues selon le procédé suivant l'une des revendications 1 à 7 à la préparation de détergents, caractérisée en ce qu'on introduit dans lesdits détergents de 1 à 30% en poids de compositions obtenues par le procédé selon l'une des revendications 1 à 7.

9. Application selon la revendication 8 caractérisée en ce que les détergents contiennent, en outre, du perborate de sodium.

10. Application selon la revendication 9 caractérisée en ce que le perborate de sodium est présent en une quantité comprise entre 2 et 15% en poids par rapport au détergent.

**Patentansprüche**

1. Verfahren zur Herstellung von Hydroperoxide von Alkalimetall-Mono- oder Polyalkylarylsulfonaten enthaltenden Verbindungen in wäßrigem Milieu, dadurch gekennzeichnet, daß

a) in einer ersten Stufe ein Alkalimetall-Mono- oder Polyalkylarylsulfonat der Formel:

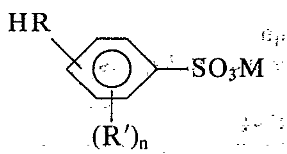

in der M ein Alkalimetall, RH ein Alkylradikal, R' ein niederes Alkylradikal bedeuten und $n \leq 4$, mit Hilfe von Sauerstoff bei einer Temperatur unterhalb 85°C und bei einem pH-Wert zwischen 9 und 10,5 in Gegenwart von 1 bis 50 ppm Kobaltionen in bezug auf das Alkalimetall-Mono- oder Polyalkylarylsulfonat behandelt wird, um eine Mischung aus

- 5 bis 65 Gew.-% nicht umgesetzten Alkalimetall-Mono- oder Polyalkylarylsulfonat,
- 25 bis 70 Gew.-% Hydroperoxid der Formel

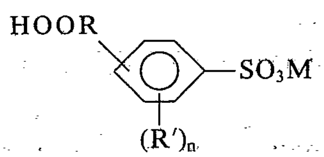

- 5 bis 30 Gew.-% Alkohol der Formel

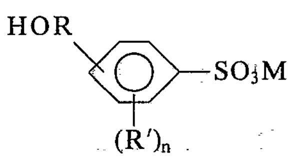

zu bilden, und

b) in einer zweiten Stufe die in der ersten Stufe erhaltene Mischung bei einem PH-Wert zwischen 4 und 10 und einer Temperatur unterhalb 50°C in Kontakt mit Wasserstoffperoxid gebracht wird, wobei die Menge an Wasserstoffperoxid zwischen 2 und 15 Gewichtsteilen (gerechnet an 100%igem Wasserstoffperoxid) für 100 Gewichtsteile der in der ersten Stufe erhaltenen Mischung liegt, und danach das so behandelte Produkt bei einer Temperatur unterhalb 50°C getrocknet wird.

2. Verfahren nach Anspruch 1, dadurch gekennzeichnet, daß das Kobaltion durch Kobaltnaphthenat zur Verfügung gestellt wird.

3. Verfahren nach einem der vorstehenden Ansprüche, dadurch gekennzeichnet, daß die Behandlung in der ersten Stufe mit Hilfe von Luft durchgeführt wird.

4. Verfahren nach einem der vorstehenden Ansprüche, dadurch gekennzeichnet, daß die zweite Verfahrensstufe an einer 30 bis 70 Gew.-% der in der ersten Stufe erhaltenen Mischung enthaltenden wäßrigen Lösung durchgeführt wird.

5. Verfahren nach einem der vorstehenden Ansprüche, dadurch gekennzeichnet, daß die Trocknung des erhaltenen Produktes unter einem reduzierten Druck zwischen 2 und 100 mm Hg durchgeführt wird.

6. Verfahren nach einem der vorstehenden Ansprüche, dadurch gekennzeichnet, daß die zweite Stufe in Gegenwart von Natriumtripolyphosphat durchgeführt wird.

7. Verfahren nach einem der vorstehenden Ansprüche, dadurch gekennzeichnet, daß das Natriummono- oder -polyalkylarylsulfonat Natriumisopropylbenzosulfonat ist.

8. Anwendung von nach dem Verfahren nach einem der Ansprüche 1 bis 7 erhaltenen Verbindungen bei der Herstellung von Detergentien, dadurch gekennzeichnet, daß man den genannten Detergentien 1 bis 30 Gew.-% der nach dem Verfahren nach einem der Ansprüche 1 bis 7 erhaltenen Verbindungen zugibt.

9. Anwendung nach Anspruch 8, dadurch gekennzeichnet, daß die Detergentien weiters Natriumperborat enthalten.

10. Anwendung nach Anspruch 9, dadurch gekennzeichnet, daß das Natriumperborat in einer Menge zwischen 2 und 15 Gew.-% in bezug auf das Detergens vorliegt.

## Claims

1. Process for the manufacture, in an aqueous medium, of compositions containing hydroperoxides of alkali metal monoalkylarylsulphonates or polyalkylarylsulphonates, characterised in that:

a) in a first step, an alkali metal monoalkylarylsulphonate or polyalkylarylsulphonate of the formula:

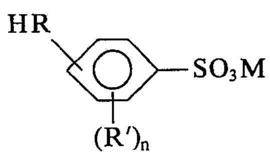

in which:
M ist an alkali metal, RH is an alkyl radical, R′ is a lower alkyl radical and $n \leq 4$,
is treated with oxygen at a temperature below 85° C, at a pH of between 9 and 10.5 and in the presence of 1 to 50 ppm of cobalt ions, relative to the alkali metal monoalkylarylsulphonate or polyalkylarylsulphonate, to form a mixture consisting, by weight, of:

- 5 to 65% of unconverted alkali metal monoalkylarylsulphonate or polyalkylarylsulphonate,
- 25 to 70% of the corresponding hydroperoxide of the formula:

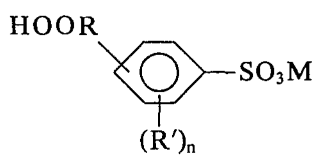

and

- 5 to 30% of the corresponding alcohol of the formula:

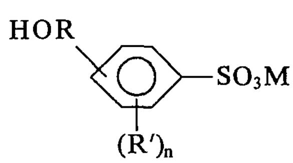

and

b) in a second step, the mixture obtained in the first step is brought into contact with hydrogen peroxide, the amount of hydrogen peroxide being between 2 and 15 parts by weight (calculated as 100% strength hydrogen peroxide) per 100 parts by weight of the mixture obtained in the first step, at a pH of between 4 and 10 and at a temperature below 50° C, and the procuct treated in thin way is then dried at a temperature below 50° C.

2. Process according to Claim 1, characterised in that the cobalt ions are provided by cobalt naphthenate.

3. Process according to one of the preceding claims, characterised in that the treatment according to the first step is carried out using air.

4. Process according to one of the preceding claims, characterised in that the second step of the process is carried out on an aqueous solution containing from 30 to 70% by weight of the mixture obtained in the first step.

5. Process according to one of the preceding claims, characterised in that the product obtained is dried under a reduced pressure of between 2 and 100 mm of mercury.

6. Process according to one of the preceding claims, characterised in that the second step is carried out in the presence of sodium tripolyphosphate.

7. Process according to one of the preceding claims, characterised in that the sodium monoalkylarylsulphonate or polyalkylarylsulphonate is sodium isopropylbenzenesulphonate.

8. Application of the compositions obtained by the process according to one of Claims 1 to 7 to the preparation of detergents, characterised in that from 1 to 30% by weight of compositions obtained by the process according to one of Claims 1 to 7 is introduced into the said detergents.

9. Application according to Claim 8, characterised in that the detergents also condtain sodium perborate.

10. Application according to Claim 9, characterised in that the sodium perborate is present in an amount of between 2 and 15% by weight, relative to the detergent.